# EUROPEAN PATENT APPLICATION

(11) **EP 4 438 612 A1**
(43) Date of publication of application: **02.10.2024**
(21) Application number: 22896935.8
(22) Date of filing: 06.01.2022
(51) Int. Cl.: C07F 9/6524, A61K 51/04, A61P 35/04

(54) **RADIOACTIVE MARKER, AND PRECURSOR COMPOUND THEREOF, PREPARATION METHOD THEREFOR, AND APPLICATION THEREOF**

(30) Priority: 26.11.2021 CN 202111419244
(71) Applicant: The Affiliated Hospital of Southwest Medical University, Luzhou, Sichuan 646000 (CN)
(72) Inventor: CHEN, Yue, Luzhou, Sichuan 646000 (CN); WANG, Yingwei, Luzhou, Sichuan 646000 (CN); WANG, Qixin, Luzhou, Sichuan 646000 (CN); QIU, Lin, Luzhou, Sichuan 646000 (CN); FENG, Yue, Luzhou, Sichuan 646000 (CN); WANG, Li, Luzhou, Sichuan 646000 (CN); CHEN, Zan, Luzhou, Sichuan 646000 (CN); YANG, Jian, Luzhou, Sichuan 646000 (CN); PENG, Dengsai, Luzhou, Sichuan 646000 (CN); LIU, Guangfu, Luzhou, Sichuan 646000 (CN); XU, Tingting, Luzhou, Sichuan 646000 (CN); XING, Naiguo, Luzhou, Sichuan 646000 (CN); LIU, Hanxiang, Luzhou, Sichuan 646000 (CN)
(74) Representative: Meissner Bolte Partnerschaft mbB
(86) International application number: PCT/CN2022/070444
(87) International publication number: WO 2023/092830

(57) **Abstract**

A precursor compound of a radioactive marker as shown in Formula (I), a radioactive marker thereof, or a pharmaceutically acceptable salt thereof. The radioactive marker is high in water solubility, good in in-vitro stability at room temperature, and high in plasma protein binding rate, and shows high and long-time bone uptake in both in-vivo distribution in mice and development of New Zealand rabbits, and animal experiments show that the development effect is better than that of ^{99m}Tc-MDP.

## Description

### Technical Field

The present disclosure belongs to the technical field of nuclear medicine, and specifically relates to radiolabeled compounds, precursor compounds thereof, preparation methods thereof and applications thereof.

### Background Art

Bone is a common site for distant metastasis of malignant tumors, and its incidence is second only to the lung and liver. Early diagnosis and treatment of metastatic bone tumors can effectively improve the prognosis and life quality of patients.

Current treatments for metastatic bone tumors mainly include radiotherapy, chemotherapy, radionuclide therapy, bisphosphonate therapy, analgesic therapy and palliative surgery. Targeted radionuclide therapy for metastatic bone tumors can significantly relieve bone pain, kill tumor cells, has few toxic side effects, and thus is a safe and effective treatment method. Currently, radiopharmaceuticals commonly used in China to treat metastatic bone tumors include ⁸⁹SrCl₂ and ¹⁵³Sm-EDTMP. They can significantly relieve bone pain and reduce the incidence of skeletal-related events. However, both of them need to be produced by nuclear reactors and are relatively expensive, and it is difficult for ⁸⁹SrCl₂ to be used for imaging and thus ⁸⁹SrCl₂ is subjected to certain use restrictions. There is an urgent clinical need for more effective, inexpensive, and easily available therapeutic radiopharmaceuticals.

⁶⁸Ga, ¹⁷⁷Lu, ²²⁵ Ac, ⁶⁴Cu, and ²²¹ At are all medical isotopes with excellent nuclear properties. ⁶⁸Ga is obtained through a ⁶⁸Ge/⁶⁸Ga generator, which is convenient in source and low in cost; it can emit β⁺ rays and is used for PET imaging. ¹⁷⁷Lu, ²²⁵Ac, ⁶⁴Cu, and ²²¹ At are all purchased from abroad, and the supply channels are smooth and stable. ¹⁷⁷Lu has a half-life (T1/2) of 6.7d, emits three kinds of β-particles with energy of 497keV (78.6%), 384keV (9.1%), and 176keV (12.2%), respectively, which can be used for treatment, and also emits γ rays of 113keV (6.4% ), and 208keV (11%), which are suitable for in vivo localization and imaging. ²²⁵Ac emits a-rays, where α-ions have higher linear energy transfer, high ray energy, short range, and thus have higher relative biological effect and the strongest killing effect on tumor cells. ²²⁵ Ac has a half-life (T1/2) of 9.9d, and its daughter nuclide ²¹³Bi has a half-life (T1/2) of 46 minutes. ⁶⁴Cu nuclide has become a research hot spot in the fields of PET molecular probes and theranostic medicine due to its properties such as suitable half-life (12.7 h), unique decay properties (β⁺ decay, β⁻ decay, electron capture), and ability to coordinate with a variety of ligands to form complexes. α-rays released by the decay of ²¹¹At have an average energy of 6.8MeV, and have a range of 55-88µm in tissues. ²¹¹At has a half-life of 7.2h and has strong clinical application value. Currently, ^{99m}Tc-MDP is the most commonly used clinical bone imaging agent, but as a single-photon imaging agent, its abilities of localization and display of lesions are much lower than those of positron imaging agents. Phosphonate (HEDP) is a drug commonly used in clinical research for imaging and targeted therapy of metastatic bone tumors. However, as a first-generation nitrogen-free bisphosphonate, HEDP has effect significantly lower than that of the second- and third-generation nitrogen-containing bisphosphonates, such as alendronic acid and ibandronic acid. Therefore, providing drugs with better imaging effects, better therapeutic effects, and abilities of both imaging and treatment of metastatic bone tumors has become an urgent problem for those skilled in the art.

### Contents of the present disclosure

A first object of the present disclosure is to provide a precursor compound represented by Formula I, which after being nuclide labeled, can produce a drug suitable for both imaging and treating a metastatic bone tumor.

A second object of the present disclosure is to provide a radiolabeled compound represented by Formula II, which is obtained by labeling a compound represented by Formula I with a nuclide.

A third object of the present disclosure is to provide a method for preparing a precursor compound represented by Formula I.

A fourth object of the present disclosure is to provide a method for preparing a radiolabeled compound represented by Formula II.

A fifth object of the present disclosure is to provide an application of a precursor compound represented by Formula I.

A sixth object of the present disclosure is to provide an application of a radiolabeled compound represented by Formula II.

In order to achieve the above objects, the technical solutions adopted by the present disclosure are as follows:
The present disclosure provides a precursor compound of a radiolabeled compound with a structure represented by Formula I or a pharmaceutically acceptable salt thereof,

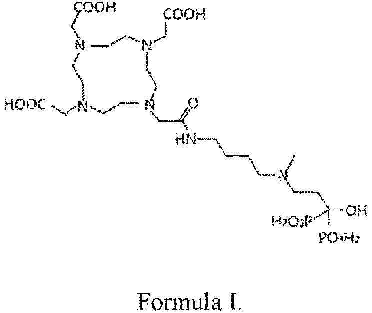

The present disclosure provides a radiolabeled compound of a compound represented by Formula I or a pharmaceutically acceptable salt thereof, which has a structure represented by Formula II, 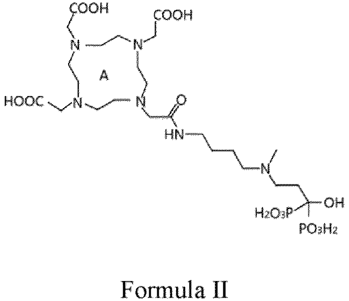 wherein, A represents a nuclide, preferably ⁶⁸Ga, ¹⁷⁷Lu, ²²⁵Ac, ⁶⁴Cu, or ²²¹At.

The present disclosure inventively binds the chelating agent DOTA with ibandronic acid to form a new compound, DOTA-ibandronic acid. When being labeled with radionuclides ⁶⁸Ga, ¹⁷⁷Lu, ²²⁵ Ac, ⁶⁴Cu, or ²¹¹At, the amount of precursor used is much lower than that of ibandronic acid alone. We provide a variety of radionuclide-labeled DOTA-ibandronic acids, which are new drugs for both imaging and treatment of metastatic bone tumors. This kind of drugs can better exert the imaging and treatment effects of radionuclides ⁶⁸Ga, ¹⁷⁷Lu, ²²⁵ Ac, ⁶⁴Cu, ²¹¹At and DOTA-ibandronic acid on metastatic bone tumors, thereby creating a synergistic effect of 1 plus 1 being greater than 2.

In some embodiments of the present disclosure, the radionuclide-labeled DOTA-ibandronic acids have a radiochemical purity of greater than or equal to 95%.

The present disclosure provides a preparation method of the precursor compound of the radiolabeled compound represented by Formula I, which comprises the following steps:
S 1, reacting Compound 1 with tert-butyl acrylate to form Compound 2;
S2, adding HCl/EtOAc dropwise to an EtOAc solution of Compound **2,** reacting to generate Compound **3;**
S3, adding Compound **3** to PhCl, adding POCl₃ and H₃PO₃ under nitrogen protection, heating and reacting, cooling and then pouring out chlorobenzene solution, adding H₂O, heating and stirring to generate Compound **4;**
S4, adding a DOTA-NHS-ester solution to an aqueous solution of Compound **4,** then adding triethylamine, and reacting to generate a compound represented by Formula I;
the reaction scheme being as follows:

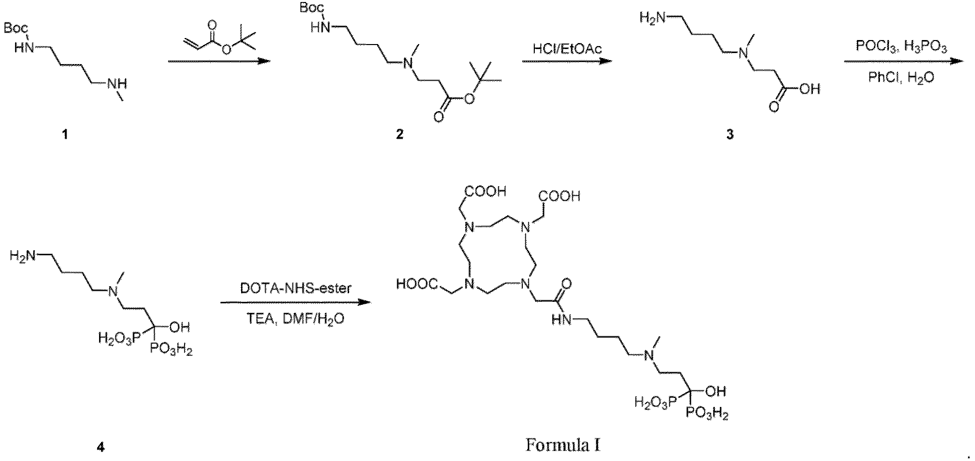

The present disclosure provides a preparation method of the radiolabeled compound represented by Formula II which comprises: reacting a compound represented by Formula I

In some embodiments of the present disclosure, the radiolabeled compound is obtained by, when the radionuclide is ⁶⁸Ga, ¹⁷⁷Lu or ⁶⁴Cu, mixing a solution of the compound represented by Formula I, a sodium acetate solution, and a radionuclide salt solution evenly, adjusting pH value of the mixed solution, reacting, adjusting pH value again, sterilizing, and filtering;
the radiolabeled compound is obtained by, when the radionuclide is ²²⁵ Ac, mixing a solution of the compound represented by Formula I, a sodium citrate solution, a sodium ascorbate solution, and a radionuclide salt solution evenly, adjusting pH value of the mixed solution, reacting, adjusting pH value again, sterilizing, and filtering;
the radiolabeled compound is obtained by, when the radionuclide is ²¹¹At, mixing a solution of the compound represented by Formula I, a sodium borate solution, and a radionuclide salt solution evenly, adjusting pH value of the mixed solution, reacting, adjusting pH value again, sterilizing, and filtering.

In some embodiments of the present disclosure, the radiolabeled compound is obtained by, when the radionuclide is ⁶⁸Ga, adding 20-30 µg of the compound represented by Formula I to 0.8-1.4 ml of a sodium acetate solution with a concentration of 0.25M; then adding a ⁶⁸Ga salt solution with an activity of 20 mCi; mixing evenly, adjusting pH value of the mixed solution to 4-7, preferably 5; reacting at 80-100°C, preferably 95°C; the reaction time being 10-30min, preferably 15min; after the reaction, adjusting the pH value to 5; in which the solution of the compound represented by Formula I has a concentration of 1 mg/ml; and the ⁶⁸Ga salt solution has a concentration of 5mCi/ml to 10mCi/ml;
the radiolabeled compound is obtained by, when the radionuclide is ¹⁷⁷Lu, adding 20-30 µg of the compound represented by Formula I to 0.8-1.4 ml of a sodium acetate solution with a concentration of 0.25M; then adding a ¹⁷⁷Lu salt solution with an activity of 2 mCi; mixing evenly, adjusting pH value of the mixed solution to 4-7, preferably 5; reacting at 80-100°C, preferably 95°C; the reaction time being 10-30min, preferably 15min; after the reaction, adjusting the pH value to 4.5; in which the solution of the compound represented by Formula I has a concentration of 1 mg/ml; and the ¹⁷⁷Lu salt solution has a concentration of 10mCi/ml to 20mCi/ml;
the radiolabeled compound is obtained by, when the radionuclide is ²²⁵ Ac, adding 20-30 µg of the compound represented by Formula I to 0.8-1.4 ml of a sodium ascorbate solution with a concentration of 0.1M and 0.8-1.4 ml of a sodium citrate solution with a concentration of 0.1M; then adding a ²²⁵ Ac salt solution with an activity of 0.01 mCi; mixing evenly, adjusting pH value of the mixed solution to 4-7, preferably 5; reacting at 80-100°C, preferably 95°C; the reaction time being 10-30min, preferably 15min; after the reaction, adjusting the pH value to 4.5; in which the solution of the compound represented by Formula I has a concentration of 1 mg/ml; and the ²²⁵ Ac salt solution has a concentration of 0.01mCi/ml to 0.02mCi/ml;
the radiolabeled compound is obtained by, when the radionuclide is ⁶⁴Cu, adding 20-30 µg of the compound represented by Formula I to 0.8-1.4 ml of a sodium acetate solution with a concentration of 0.25M; then adding a ⁶⁴Cu salt solution with an activity of 5mCi; mixing evenly, adjusting pH value of the mixed solution to 4-7, preferably 5; reacting at 80-100°C, preferably 95°C; the reaction time being 10-30min, preferably 15min; after the reaction, adjusting the pH value to 4; in which the solution of the compound represented by Formula I has a concentration of 1 mg/ml; and the ⁶⁴Cu salt solution has a concentration of 5mCi/ml to 10mCi/ml;
the radiolabeled compound is obtained by, when the radionuclide is ²¹¹ At, adding 20-30 µg of the compound represented by Formula I to 0.8-1.4 ml of a sodium borate solution with a concentration of 0.25M; then adding a ²¹¹ At salt solution with an activity of 1mCi; mixing evenly, adjusting pH value of the mixed solution to 4-7, preferably 5; reacting at 80-100°C, preferably 95°C; the reaction time being 10-30min, preferably 15min; after the reaction, adjusting the pH value to 7; in which the solution of the compound represented by Formula I has a concentration of 1 mg/ml; and the ²¹¹At salt solution has a concentration of 1.0mCi/ml to 2.0mCi/ml.

The present disclosure provides a use of the compound represented by Formula I in the manufacture of a medicament for both imaging and treating a metastatic bone tumor.

The present disclosure provides a use of the compound represented by Formula II in the manufacture of a medicament for both imaging and treating a metastatic bone tumor.

Compared with the prior art, the present disclosure has the following advantageous effects:
The present disclosure provides a scientific design and a simple method. The present disclosure inventively binds ibandronic acid with a chelating agent to obtain a DOTA-ibandronic acid, and then labels the DOTA-ibandronic acid with radionuclide ⁶⁸Ga, ¹⁷⁷Lu, ²²⁵ Ac, ⁶⁴Cu, or ²¹¹At to obtain ⁶⁸Ga-, ¹⁷⁷Lu-, ²²⁵Ac^{_}, ⁶⁴Cu-, or ²¹¹At-DOTA-ibandronic acid. The ⁶⁸Ga-, ¹⁷⁷Lu-, ²²⁵Ac-, ⁶⁴Cu-, or ²¹¹At-DOTA-ibandronic acid of the present disclosure has great water solubility, good in vitro stability at room temperature, high plasma protein binding rate, and high and long bone uptake as shown by the in vivo distribution in mice and the imaging of New Zealand rabbits, so it is a bone imaging agent with excellent performance and a radioactive therapeutic agent for metastatic bone tumors. The pharmacodynamic experiments show that ⁶⁸Ga-, ¹⁷⁷Lu-, ²²⁵Ac-, ⁶⁴Cu-, or ²¹¹At-DOTA-ibandronic acid has better effect on either diagnosis or treatment than the corresponding group of ⁶⁸GaCl₃, ¹⁷⁷LuCl₃, ²²⁵AcCl₃, ⁶⁴CuCl₂, Na²¹¹At or DOTA-ibandronic acid, indicating the feasibility and effectiveness of ⁶⁸Ga-, ¹⁷⁷Lu-, ²²⁵Ac-, ⁶⁴Cu-, or ²¹¹At-DOTA-ibandronic acid in the diagnosis and treatment of malignant tumor bone metastasis.

The labeling method of the present disclosure for preparing ⁶⁸Ga-, ¹⁷⁷Lu-, ²²⁵Ac-, ⁶⁴Cu-, or ²¹¹At-DOTA-ibandronic acid is simple, where the reaction time is short, the labeling yield is high, and the amount of precursor used is very small (microgram level). The ratio of targeted lesions to non-targeted tissue (T/N value) for the labeled compounds is very high, up to 10 times or more. According to literature reports, there is high therapeutic potential value when the T/N value is greater than 4-5.

### Brief Description of the Drawings

Figure 1 shows the PET imaging of ⁶⁸Ga-DOTA-IBA New Zealand rabbits at 1h and 3h in Experimental Example 5;
Figure 2 shows the SPECT whole-body static imaging of ¹⁷⁷Lu-DOTA-IBA New Zealand rabbits on 1d, 3d, 5d, and 7d in Experimental Example 6;
Figure 3 shows the SPECT whole-body static imaging of ²²⁵Ac-DOTA-IBA New Zealand rabbits on 1d, 3d, and 5d in Experimental Example 7;
Figure 4 shows the HPLC diagram of the precursor compound represented by Formula I;
Figure 5 shows the LC-MS diagram of the precursor compound represented by Formula I.

### Specific Models for Carrying Out the present disclosure

In order to make the objectives, technical solutions, and advantages of the examples of the present disclosure clearer, the technical solutions in the examples of the present disclosure will be described clearly and completely below. If the specific conditions are not specified in the examples, the conditions should be carried out according to the conventional conditions or the conditions recommended by the manufacturer. If the manufacturers of the reagents or instruments used are not indicated, the reagents or instruments are all conventional products that can be purchased commercially.

### Example 1

In this example, the preparation method of the precursor compound represented by Formula I of the present disclosure was disclosed, and its synthesis route was:

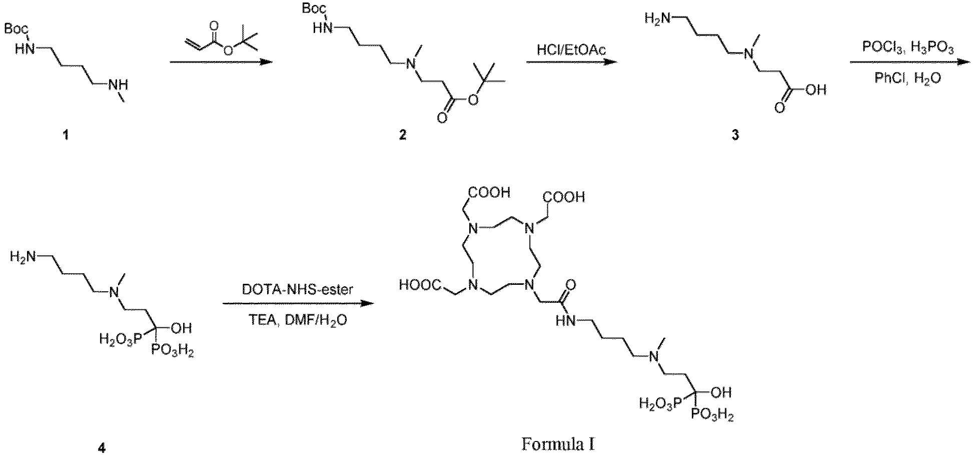

### S1. Preparation of Compound 2

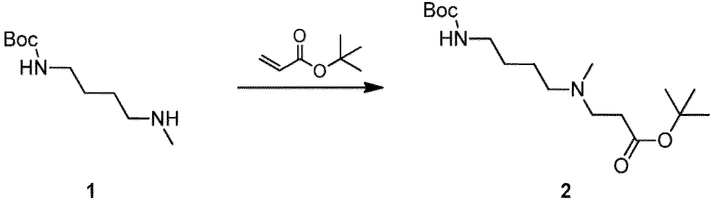

Tert-butyl acrylate (6.34 g, 49.4 mmol) was added to Compound **1** (5.00 g, 24.7 mmol). The reaction was performed under stirring at 25°C for 3 hours. TLC was used to monitor that the reaction was completed and a new spot was generated. The reaction solution was concentrated to obtain colorless oily Compound **2** (7.90 g, yield: 96.7%). The crude product was used directly in the next reaction without purification.

### S2. Preparation of Compound 3

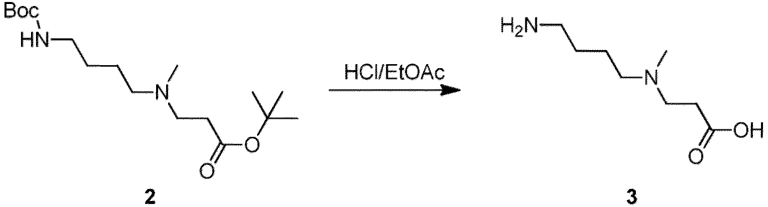

HCl/EtOAc (4 M, 20.0 mL) was added dropwise to an EtOAc (2.00 mL) solution of Compound **2** (1.90 g, 5.75 mmol) under ice bath conditions. The reaction solution was stirred at 25°C for 2 hours. LC-MS detected that the reaction of raw material was completed. The reaction solution was concentrated under vacuum to obtain colorless oily crude Compound **3** (800 mg, yield: 79.9%), which was directly used in the next reaction without further purification.

### S3. Preparation of Compound 4

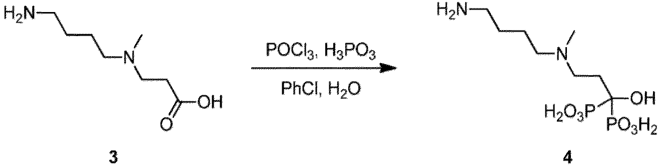

Compound **3** (800 mg, 3.24 mmol, HCl salt) was added to PhCl (100 mL), and POCl₃ (9.93 g, 64.7 mmol) and H₃PO₃ (5.31 g, 64.7 mmol) were added under nitrogen protection. The reaction mixture was heated to 80°C and stirred for 16 hours. After cooling, the chlorobenzene solution was poured out, H₂O (20.0 mL) was added, and stirred at 80°C for 5 hours. LC-MS monitored the existence of mass of product. The reaction solution was filtered and concentrated under vacuum, and the residue was purified through prep-HPLC (YMC-Actus Triart Diol-HILIC, 150*30 mm, 5 µm, 120Å Mobile phase: 0.2% CH₃COOH, CH₃CN) to obtain Compound **4** as a white solid (90.0 mg, 8.68% yield)

### S4. Preparation of compound Carbs represented by Formula I

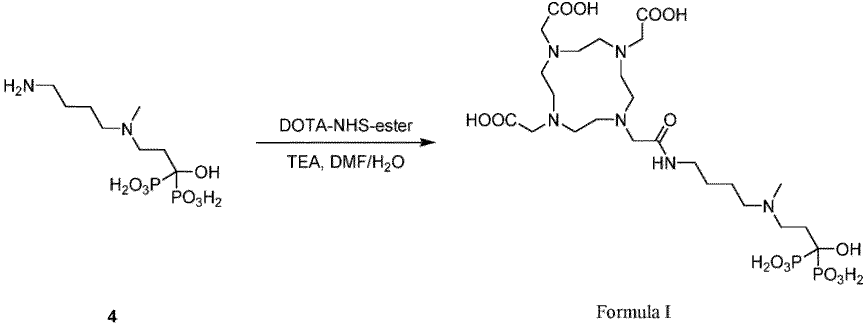

ADMF (200 µL) solution of DOTA-NHS-ester (141 mg, 281 µmol) was added to a H₂O (200 µL) solution of Compound **4** (60.0 mg, 187 µmol), then triethylamine (114 mg, 1.12 mmol) was added. The reaction solution was stirred at 25°C for 16 hours. LC-MS monitored the existence of mass of product. The reaction solution was filtered and concentrated, and the residue was purified by prep-HPLC (Hilic, 0.2% CH₃COOH) to obtain the compound Carbs represented by Formula I as a white solid (4.00 mg, 3.02% yield, 86.8% purity).

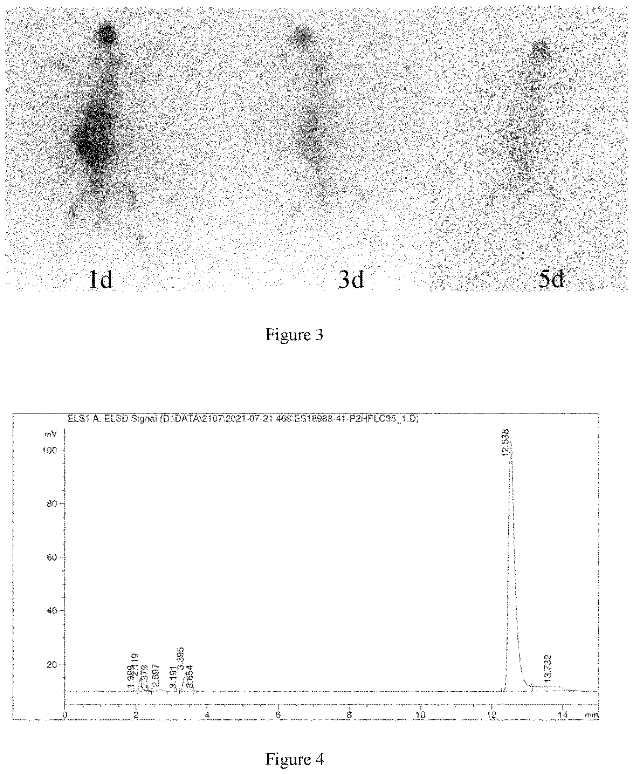

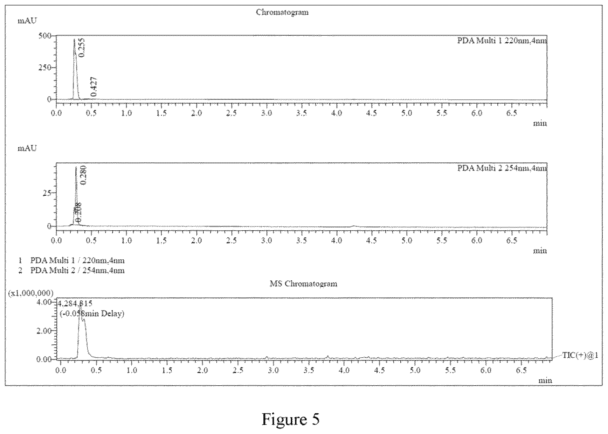

The HPLC diagram of the compound represented by Formula I was shown in Figure 4, and the LC-MS diagram was shown in Figure 5.

### Example 2

In this example, the amount of DOTA-ibandronic acid for preparing ⁶⁸Ga-DOTA-ibandronic acid was investigated, and specified as follows:
Eight EP tubes were taken, and added with 5 µg, 10 µg, 15 µg, 20 µg, 25 µg, 30 µg, 35 µg, and 40 µg of DOTA-ibandronic acid (1mg/ml), respectively. To each tube, 1mL of sodium acetate solution (0.25M) was added, and then 4ml of freshly rinsed ⁶⁸GaCl₃ eluate (5mci/ml) was added. The pH value was adjusted to about 5 with 0.25M sodium acetate solution and 0.01M hydrochloric acid, and the mixed solution was shaken and mixed thoroughly, and reacted in a 95°C metal bath for 15 minutes. After the reaction was completed, the reaction solution was cooled to room temperature, the pH value of each tube was adjusted to 4-6, and the reaction solution was sterilized and filtered with a 0.22 µm filter membrane. Subsequently, the radiochemical purity of ⁶⁸Ga-DOTA-ibandronic acid was determined using a thin layer paper chromatography (TLC). The results were shown in Table 1:

**Table 1: Radiochemical purity results at different amounts of DOTA-ibandronic acid**

| No. | DOTA-ibandronic acid (µg) | Radiochemical purity |
|---|---|---|
| 1 | 5 | 50% |
| 2 | 10 | 62% |
| 3 | 15 | 83% |
| 4 | 20 | 95% |
| 5 | 25 | 97% |
| 6 | 30 | 96% |
| 7 | 35 | 92% |
| 8 | 40 | 92% |

As can be seen from Table 1, when other conditions are fixed, the radiochemical purity of ⁶⁸Ga-DOTA-ibandronic acid is optimal when the amount of DOTA-ibandronic acid is 20-30 µg, and when the amount is lower or higher than this range, the radiochemical purity will gradually decrease.

### Example 3

In this example, the pH value in the present disclosure was investigated, and specified as follows:
Seven EP tubes were taken, and 25 µg of DOTA-ibandronic acid (1mg/ml), 1ml of sodium acetate solution (0.25M), and 4ml of freshly rinsed ⁶⁸GaCl₃ eluate (5mci/ml) were added to each tube in sequence. By using 0.25M sodium acetate solution and 0.1M hydrochloric acid, the pH values in these tubes were adjusted to 2, 3, 4, 5, 6, 7, and 8, respectively. The mixed solution was shaken and mixed thoroughly, and reacted in a 95°C metal bath for 15 minutes. After the reaction was completed, the reaction solution was cooled to room temperature, and sterilized and filtered with a 0.22 µm filter membrane. Subsequently, the radiochemical purity of ⁶⁸Ga-DOTA-ibandronic acid was determined using a thin layer paper chromatography (TLC). The results were shown in Table 2:

**Table 2: Radiochemical purity results at different pH values**

| No. | PH value | Radiochemical purity |
|---|---|---|
| 1 | 2 | 47% |
| 2 | 3 | 89% |
| 3 | 4 | 92% |
| 4 | 5 | 98% |
| 5 | 6 | 95% |
| 6 | 7 | 27% |
| 7 | 8 | 18% |

It could be seen from Table 2 that the radiochemical purity of DOTA-ibandronic acid is optimal when the pH value is 5. The radiochemical purity decreases gradually when the pH value is higher or lower than 5. The radiochemical purity decreases quickly when the pH value is higher than 6.

### Example 4

In this example, the amount of sodium acetate in the present disclosure was investigated, and specified as follows:
Eight EP tubes were taken, and added with 0.2ml, 0.4ml, 0.6ml, 0.8ml, 1.0ml, 1.2ml, 1.4ml, and 1.6ml of sodium acetate solution (0.25M), respectively. To each tube, 25 µg of DOTA-ibandronic acid (1mg/ml) and 4ml of freshly rinsed ⁶⁸GaCl₃ eluate (5mci/ml) were added, and the pH value was adjusted to 5 with 0.25M sodium acetate solution and 0.1M hydrochloric acid, respectively. The mixed solution was shaken and mixed thoroughly, and reacted in a 95°C metal bath for 15 minutes. After the reaction was completed, the reaction solution was cooled to room temperature, the pH value of each tube was adjusted to 5, and the reaction solution was sterilized and filtered using a 0.22 µm filter membrane. Subsequently, the radiochemical purity of ⁶⁸Ga-DOTA-ibandronic acid was determined using a thin layer paper chromatography (TLC). The results were shown in Table 3 below:

**Table 3: Radiochemical purity results at different amounts of sodium acetate solution (0.25M)**

| No. | Sodium acetate solution (ml) | Radiochemical purity |
|---|---|---|
| 1 | 0.2 | 60% |
| 2 | 0.4 | 66% |
| 3 | 0.6 | 85% |
| 4 | 0.8 | 92% |
| 5 | 1 | 96% |
| 6 | 1.2 | 94% |
| 7 | 1.4 | 91% |
| 8 | 1.6 | 84% |

As can be seen from Table 3, when other conditions remain unchanged, the radiochemical purity of ⁶⁸Ga-DOTA-ibandronic acid is optimal when the amount of sodium acetate solution is 0.8-1.4ml, and when the amount is either lower or higher than this range, radiochemical purity will gradually decrease.

### Example 5

In this example, the reaction temperature and time duration in the present disclosure were investigated, and specified as follows:
Eighteen EP tubes were taken, and divided into three groups: Group A (room temperature: 25±2°C), Group B (60°C), and Group C (95°C). Six tubes of each group were taken and marked as A/B/C_{①-⑥}. To each tube, 25 µg of DOTA-ibandronic acid (1mg/ml), 1ml of sodium acetate solution (0.25M) and 4ml of freshly rinsed ⁶⁸GaCl₃ eluate (5mci/ml) were added sequentially in advance, and the pH value was adjusted to about 5 with 0.25M sodium acetate solution and 0.1M hydrochloric acid, respectively. The mixed solution was shaken and mixed thoroughly, then the 6 tubes of each group were subjected to reaction for 5min, 10min, 15min, 20min, 25min, and 30min, respectively, at the temperature of each group. After the reaction was completed, the reaction solution was cooled to room temperature, the pH value of each tube was adjusted to 5, and the reaction solution was sterilized and filtered with a 0.22 µm filter membrane. Subsequently, the radiochemical purity of ⁶⁸Ga-DOTA-ibandronic acid was determined using a thin layer paper chromatography (TLC). The results were shown in Table 4 below:

**Table 4: Radiochemical purity results for different temperatures and time durations**

| Temperature/ time duration | 5min | 10min | 15min | 20min | 25min | 30min |
|---|---|---|---|---|---|---|
| 25±2°C | 40% | 52% | 56% | 65% | 70% | 72% |
| 60°C | 56% | 65% | 79% | 81% | 84% | 85% |
| 95°C | 78% | 87% | 96% | 94% | 94% | 93% |

It could be seen from Table 4 that when other conditions remain unchanged, the radiochemical purity of ⁶⁸Ga-DOTA-ibandronic acid is optimal when the reaction is carried out at 95°C for 15 minutes. When the reaction time is too long, too short, or the temperature is too low, the radiochemical purity will decrease.

### Example 6

In this example, the preparation method of ¹⁷⁷Lu-DOTA-ibandronic acid of the present disclosure was disclosed, and specified as follows:
To DOTA-ibandronic acid (25µg), 1ml of sodium acetate solution (0.25M) and then 0.2ml of freshly rinsed ¹⁷⁷LuCl₃ eluate (about 2.0mci) were added. The mixed solution was adjusted to a pH value of about 5 with 0.25M sodium acetate solution and 0.01M hydrochloric acid, and subjected to reaction in a 95°C metal bath for 15 minutes. The reaction solution was taken out and adjusted to have a pH of about 4.5 with 0.04M hydrochloric acid. Then the reaction solution was passed through a CM column (to remove free ¹⁷⁷Lu), and then the product was passed through a sterile filter membrane, to finally obtain ¹⁷⁷Lu-DOTA-ibandronic acid.

### Example 7

In this example, a new preparation method of ²²⁵Ac-DOTA-ibandronic acid of the present disclosure was disclosed, and specified as follows:
To DOTA-ibandronic acid (25µg), 1ml of ascorbic acid solution (0.1M) and 1ml of sodium citrate solution (0.1M), then 100µl of freshly rinsed ²²⁵AcCl₃ eluate (about 0.01mci) were added. The mixed solution was adjusted to a pH of about 4.5 with 0.04M hydrochloric acid, and subjected to reaction in a 95°C metal bath for 15 minutes. The reaction solution was taken out and adjusted to have a pH of about 4.5 with 0.04M hydrochloric acid, and then the product was filtered with sterile filtration membrane to finally obtain ²²⁵Ac-DOTA-ibandronic acid.

### Example 8

In this example, a new preparation method of ⁶⁴Cu-DOTA-ibandronic acid of the present disclosure was disclosed, and specified as follows:
To DOTA-ibandronic acid (25µg), 1ml of sodium acetate solution (0.25M) and then 1ml of freshly rinsed ⁶⁴CuCl₂ eluate (about 5mci) were added. The mixed solution was adjusted to a pH of about 4 with 0.04M hydrochloric acid, and subjected to reaction in a 95°C metal bath for 15 minutes. The reaction solution was taken out and adjusted to have a pH of about 4 with 0.04M hydrochloric acid, and then the product was passed through a sterile filter membrane to finally obtain ⁶⁴Cu-DOTA-ibandronic acid.

### Example 9

In this example, a new preparation method of ²¹¹At-DOTA-ibandronic acid of the present disclosure was disclosed, and specified as follows:
To DOTA-ibandronic acid (25µg), 1ml of sodium borate solution (0.25M) and then 1ml of freshly rinsed Na²¹¹ At eluate (about 1.0mci) were added. The mixed solution was adjusted to a pH of about 7, and subjected to reaction in a metal bath at 95°C for 15 minutes. The reaction solution was adjusted to have a pH of about 7, and then the product was passed through a sterile filter membrane to finally obtain ²¹¹At-DOTA-ibandronic acid.

In Experimental Examples 1-5, the ⁶⁸Ga-DOTA-ibandronic acid used was prepared according to the No.5 method in Table 1 of Example 2.

### Experimental Example 1

In this experimental example, the in vitro stability test of ⁶⁸Ga-DOTA-ibandronic acid of the present disclosure was disclosed, and specified as follows:
Four EP tubes were taken and marked as numbers ① to ④ in sequence, in which 0.1 ml of normal saline was added to numbers ① and ②, respectively, 0.1 ml of fresh human serum diluted 10 times was added to numbers ③ and ④, respectively, and then 1.0 mci of freshly prepared ⁶⁸Ga-DOTA-ibandronic acid was added to these 4 tubes, respectively. Numbers ① and ③ were placed at room temperature (25±2°C) and numbers ② and ④ were placed in a 37°C metal bath for incubation. The radiochemical purity values of numbers ① to ④ were measured using a thin layer paper chromatography (TLC) at 10min, 30min, 1h, 2h, 3h, 4h, respectively. The results were shown in Table 5 below.

**Table 5: Radiochemical purity values of ⁶⁸Ga-DOTA-ibandronic acid under different processing procedures and processing time durations**

| | 10min | 30min | 1h | 2h | 3h | 4h |
|---|---|---|---|---|---|---|
| Room temperature (normal saline) | 98% | 97% | 95% | 95% | 95% | 93% |
| Room temperature (serum) | 99% | 99% | 99% | 96% | 96% | 93% |
| 37°C (normal saline) | 99% | 96% | 96% | 92% | 90% | 87% |
| 37°C (serum) | 99% | 97% | 96% | 92% | 88% | 82% |

It can be seen from Table 5 that the ⁶⁸Ga-DOTA-ibandronic acid has good in vitro stability at room temperature, and its radiochemical purity is still >90% after being placed in normal saline and serum for 4 hours at room temperature.

### Experimental Example 2

In this experimental example, the lipid-water distribution coefficient of the ⁶⁸Ga-DOTA-ibandronic acid of the present disclosure was investigated, and specified as follows:
Three Sml centrifuge tubes were taken, and numbered as A-①, A-② and A-③, respectively. 0.5ml of n-octanol, 0.485ml of deionized water and 0.25ml of freshly prepared ⁶⁸Ga-DOTA-ibandronic acid with about 0.5mci were added to each tube, and the centrifuge tubes were shaken by a vortex mixer for 20 minutes, then centrifuged at 2000r/min for 5 minutes, from which 0.1ml of upper liquid (organic phase) was respectively collected into 3 corresponding test tubes (numbered as B-①, B-②, B-③, respectively), and 0.1 ml of lower liquid (aqueous phase) was respectively collected into 3 corresponding test tubes (numbered as C-(D, C-②, C-③, respectively). The total radioactivity count of the organic phase and that of the aqueous phase were measured by γ counter, respectively, and the lipid-water distribution coefficient IgP was calculated by formula.

The results show that the lipid-water distribution coefficient IgP of ⁶⁸Ga-DOTA-ibandronic acid of the present disclosure is -2.33, indicating that the labeled compound has high water solubility and low lipid solubility.

### Experimental Example 3

In this experimental example, the plasma protein binding rate of the ⁶⁸Ga-DOTA-ibandronic acid of the present disclosure was investigated, and specified as follows:
1 mL of fresh human plasma anticoagulated with heparin was taken for later use. Three EP tubes were taken and numbered as A-①, A-②, and A-③, and were each added with 0.1ml of fresh human plasma and 0.25ml of freshly prepared ⁶⁸Ga-DOTA-ibandronic acid with about 0.5mci. Then they were incubated in a metal bath at 37°C for 30 minutes. Three Sml centrifuge tubes were taken and numbered as B-①, B-②, and B-③. The incubated solutions were transferred to the corresponding centrifuge tubes, respectively. Then 1ml of 25% trichloroacetic acid solution was added to each centrifuge tube, mixed thoroughly, then centrifuged at 2000r/min for 5 minutes. The supernatants were respectively collected into 3 corresponding test tubes (numbered as C-①, C-②, C-③, respectively). The centrifugation and supernatant collection were repeated 3 times. The total radioactivity count of the precipitations in the centrifuge tubes and that of the supernatants in the test tubes were measured by a γ counter, respectively, and the plasma protein binding rate (PPB) was calculated.

The results show that the ⁶⁸Ga-DOTA-ibandronic acid of the present disclosure has a PPB of 81% after being incubated in plasma for 30 minutes. The high plasma protein binding rate indicates that the labeled compound will retain in the body for a long time, be less likely to be cleared, and can achieve good imaging effect.

### Experimental Example 4

In this experimental example, the in vivo distribution of the ⁶⁸Ga-DOTA-ibandronic acid of the present disclosure in mice was investigated, and specified as follows:
Twenty healthy Kunming mice (body weight of 18-22g) were taken, and randomly divided into 4 groups, 5 in each group, half of them being male and half of them being female. 0.5mci/0.1ml freshly prepared ⁶⁸Ga-DOTA-ibandronic acid was injected via tail vein. The animals were decapitated after 15 min, 30 min, 1 h, and 2 h, respectively. The carotid blood, brain, heart, liver, spleen, lung, kidney, stomach, small intestine, muscle, and femur tissues were weighed, and measured their radioactivity counts with a γ counter, from which, after time decay correction, the percent injected dose per gram of tissue (%ID/g) was calculated. The in vivo distribution results of ⁶⁸Ga-DOTA-ibandronic acid in mice were shown in Table 6 below:

**Table 6: In vivo distribution results of ⁶⁸Ga-DOTA-ibandronic acid in mice**

| Tissue or organ | %ID/g | | | | |
|---|---|---|---|---|---|
| | 15min | 30h | 1h | 2h | 4h |
| Heart | 1.037 | 0.557 | 0.349 | 0.216 | 0.159 |
| Liver | 0.526 | 0.658 | 0.498 | 0.418 | 0.325 |
| Spleen | 0.665 | 0.562 | 0.332 | 0.350 | 0.283 |
| Lung | 0.869 | 1.479 | 0.659 | 0.477 | 0.272 |
| Kidney | 4.658 | 5.391 | 3.774 | 2.414 | 1.023 |
| Stomach | 1.254 | 1.365 | 0.964 | 0.658 | 0.562 |
| Blood | 2.635 | 2.837 | 1.622 | 0.452 | 0.325 |
| Brain | 0.097 | 0.063 | 0.036 | 0.039 | 0.061 |
| Femur | 2.318 | 3.346 | 6.693 | 8.365 | 4.268 |
| Muscle | 0.472 | 0.526 | 0.365 | 0.194 | 0.856 |

It can be seen from Table 6 that the bones have a high uptake rate of ⁶⁸Ga-DOTA-ibandronic acid, reaching the maximum value (8.365 %ID/g) at 2h. After that, the uptake in bones decreases over time, but by 4h, the percent injected dose per gram of tissue in bones can still reach 4.268 %ID/g, which is significantly higher than those of other organs and tissues, indicating that the ⁶⁸Ga -DOTA-ibandronic acid has a strong effect and a long residence on bones, and thus can achieve ideal imaging effects.

### Experimental Example 5

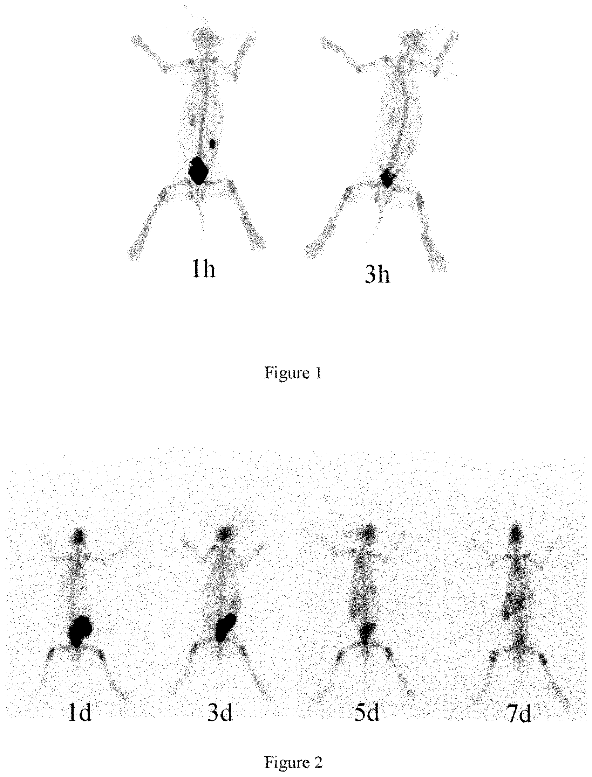

In this experimental example, the imaging of ⁶⁸Ga-DOTA-ibandronic acid of the present disclosure in New Zealand rabbits at different time points was investigated, and specified as follows:
One New Zealand rabbit of about 2Kg was taken and injected with about 1.0mci/0.2ml of freshly prepared ⁶⁸Ga-DOTA-ibandronic acid via ear vein, and PET whole-body imaging was performed at 1h and 3h after the injection, respectively (Figure 1).

It can be seen from Figure 1 that 1 hour after injection, the kidney and bladder show obvious images, the chest cavity and soft tissue show obvious images, the bones of the whole body show clear images, and the joints of the limbs show obvious images. Figure 2 shows that as time goes by, the images of the chest cavity and soft tissue gradually become weak, the image of both kidneys gradually becomes weak and gradually transfers to the bladder, the image of the bladder gradually becomes thick, and the image of the bladder gradually disappears as urine is discharged. Therefore, the ⁶⁸Ga-DOTA-ibandronic acid is an imaging agent that is cleared quickly by soft tissue, has high bone uptake, and is excreted through the urinary system, and it stays on the bone for a relatively long time. If it is applied to metastatic bone cancer patients, good imaging results will be achieved.

### Experimental Example 6

The imaging of ¹⁷⁷Lu-DOTA-ibandronic acid prepared according to the method of Example 6 in New Zealand rabbits at different time points was investigated, and specified as follows:
One New Zealand rabbit of about 2Kg was taken and injected with about 2.0mci/0.2ml of freshly prepared ¹⁷⁷Lu-DOTA-ibandronic acid via ear vein, and PET whole-body imaging was performed on 1d, 3d, 5d, and 7d after the injection, respectively (Figure 2).

### Experimental Example 7

The imaging of ²²⁵ Ac-DOTA-ibandronic acid prepared according to the method of Example 7 in New Zealand rabbits at different time points was investigated, and specified as follows:
One New Zealand rabbit of about 2Kg was taken and injected with about 0.01mci/2ml of ²²⁵Ac-DOTA-ibandronic acid freshly prepared under the above optimal labeling conditions via ear vein, and PET whole-body imaging was performed on 1d, 3d, and 5d after the injection, respectively (Figure 3).

### Experimental Example 8

In this experimental example, the therapeutic effects of ¹⁷⁷Lu-DOTA-ibandronic acid of the present disclosure on tumor-bearing mice (PC-3, H1975, MDA-MB-231 bone metastasis models) were investigated. The ¹⁷⁷Lu-DOTA-ibandronic acid used in this experimental example was prepared according to the method of Example 6.
(1) Establishment of tumor-bearing mouse models: 24 SPF male nude mice and 12 SPF female nude mice were taken, and anesthetized with isoflurane. Then the skin around the left knee joint was disinfected, and the left hind limb was bent. An insulin needle ( 29G needle) was inserted into the tibia vertically from concave point of the articular fossa of the tibia, slowly rotated and inserted into the medullary cavity, to inject 25 µl of cell culture (approximately 2×10⁶) of PC-3 (12 males), H1975 (12 males), and MDA-MB-231 (12 females). Then disinfection was carried out, and the holes were sealed with sterile bone wax. The animals were placed on a 37°C hot-plate for rewarming. After resuscitation, they were returned to the cage and continued to be raised.
(2) Testing of tumor-bearing mouse models: After 4 weeks of inoculation, 36 nude mice were scanned using Micro-CT, respectively. It was observed that the nude mice each had varying degrees of bone destruction, accompanied by varying degrees of soft tissue swelling, indicating that the bone metastasis models were successfully established.
(3) Methods of different treatments and grouping: The 36 successfully modelled tumor-bearing mice were randomly divided into 4 groups. Among them, 2 groups were injected with ¹⁷⁷Lu-DOTA-ibandronic acid with low and high radioactivity (100 µCi, 500 µCi) into the tail vein, respectively, 3 animals in each group; and the animals in the blank control groups were injected with normal saline, and divided into a small volume group and a large volume group (10 µl, 50 µl), 3 animals in each group.
(4) Detection and results of treatment effect: Free movement pain behavior scoring was carried out on days 0, 7, 14, 21, 28, and 35, and used as the main indicator of bone pain relief in the tumor-bearing mice. At the corresponding time points, the tumor-bearing mice were placed in a transparent smooth flat-bottomed plastic box. The tumor-bearing mice could move freely in the box and were observed after acclimatization for 30 minutes. The pain behavior scoring was performed according to the following standards: a score of 0, the movement was normal, and the movements of the hind limbs on the model side and the control side were the same; a score of 1, the hind limb on the model side was slightly lame; a score of 2, the degree of lameness of the hind limb on the model side was between a score of 1 and 3; a score of 3, the hind limb on the model side was severely lame; a score of 4, the hind limb on the model side was completely unable to move and cannot touch the ground. The results were shown in Table 7 below:

**Table 7: Pain behavior scores of free movement of tumor-bearing mice in each group under different treatment methods and treatment time courses**

| | Day 0 | Day 7 | Day 14 | Day 21 | Day 28 | Day 35 |
|---|---|---|---|---|---|---|
| Low activity ¹⁷⁷Lu-DOTA-ibandronic acid | (2,2,3) | (2,3,3) | (2,3,2) | (2,4,2) | (1,1,2) | (1,/,1) |
| High activity ¹⁷⁷Lu-DOTA-ibandronic acid | (2,2,2) | (2,2,3) | (2,2,3) | (2,1,2) | (2,1,/) | (1,1,/) |
| Small volume normal saline group | (1,3,4) | (2,3,4) | (3,4,/) | (3,/,/) | (/,/,/) | (/,/,/) |
| Large volume normal saline group | (4,2,3) | (/,2,3) | (/,3,4) | (/,3,/) | (/,/,^{,}/) | (/,/,/) |

| | | | | | | |
|---|---|---|---|---|---|---|
| Note: ( ) represents the 1^{st}, 2^{nd} and 3^{rd} tumor-bearing mice in each group, / represents the death of the tumor-bearing mice. | | | | | | |

It can be seen from Table 7 that, taking Day 0 as the baseline, the average decrease in free movement pain behavior scores of the surviving tumor-bearing mice at 5 weeks after treatment with the dose of high-activity ¹⁷⁷Lu-DOTA-ibandronic acid (500 µCi) is the largest among all groups, and the number of survivors is relatively the largest; the average decreases in free movement pain behavior scores of the surviving tumor-bearing mice at 5 weeks after treatment with doses of ¹⁷⁷Lu-DOTA-ibandronic acid of different activities are higher than that of the corresponding control group.

The above results show that when the tumor-bearing mice are treated with the dose of high-activity ¹⁷⁷Lu-DOTA-ibandronic acid (500 µCi), their bone pain is relieved to the greatest extent, and the survival rate within 5 weeks of administration is relatively highest, and the therapeutic efficacy of different doses of ¹⁷⁷Lu-DOTA-ibandronic acid is higher than that of the corresponding control group, indicating the feasibility and effectiveness of ¹⁷⁷Lu-DOTA-ibandronic acid in the treatment of malignant tumor bone metastasis.

Based on the above Examples 1-9 and Experimental Examples 1-8, ⁶⁸Ga/¹⁷⁷Lu/²²⁵Ac/⁶⁴Cu/²²¹At-DOTA-ibandronic acid is provided by a simple labeling method in which the reaction time is short and the labeling yield is high, and has a high water solubility, a good in vitro stability at room temperature, a high plasma protein binding rate, and shows high and long-term bone uptake in both the in vivo distribution in mice and the imaging in New Zealand rabbits, indicating that the preparation invented in the present application is a bone imaging agent with excellent performance and a radioactive drug for the treatment of metastatic bone tumors.

The above-described examples are merely a part, but not all of the examples of the present disclosure. The detailed description of the examples of the present disclosure is not intended to limit the scope of the present disclosure, but rather to represent selected examples of the present disclosure. Based on the examples of the present disclosure, all other examples obtained by those of ordinary skill in the art without making inventive efforts fall within the scope sought to be protected by the present disclosure.

## Claims

1. A precursor compound of a radiolabeled compound with a structure represented by Formula I or pharmaceutically acceptable salt thereof,

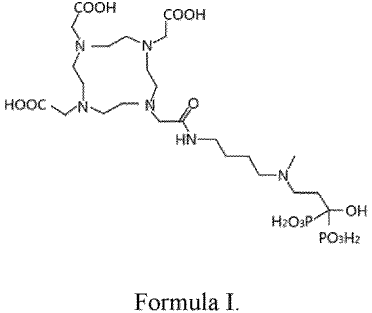

2. A radiolabeled compound of a compound represented by Formula I or pharmaceutically acceptable salt thereof, **characterized by** having a structure represented by Formula II, 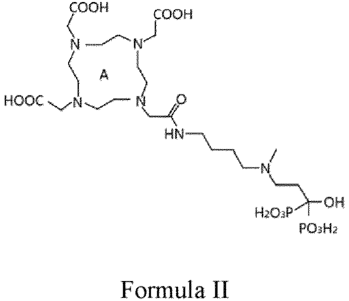 wherein, A represents a radionuclide, preferably ⁶⁸Ga, ¹⁷⁷Lu, ²²⁵ Ac, ⁶⁴Cu, or ²²¹At.

3. The radiolabeled compound or pharmaceutically acceptable salt thereof according to claim 2, **characterized by** having a radiochemical purity of greater than or equal to 95%.

4. A preparation method of the precursor compound of the radiolabeled compound according to claim 1, **characterized by** comprising the following steps:
S1, reacting Compound 1 with tert-butyl acrylate to form Compound 2;
S2, adding HCl/EtOAc dropwise to an EtOAc solution of Compound 2, reacting to generate Compound 3;
S3, adding Compound 3 to PhCl, adding POCl₃ and H₃PO₃ under nitrogen protection, heating and reacting, cooling and then pouring out chlorobenzene solution, adding H₂O, heating and stirring to generate Compound 4;
S4, adding a DOTA-NHS-ester solution to an aqueous solution of Compound 4, then adding triethylamine, and reacting to generate a compound represented by Formula I;
the reaction scheme being as follows:

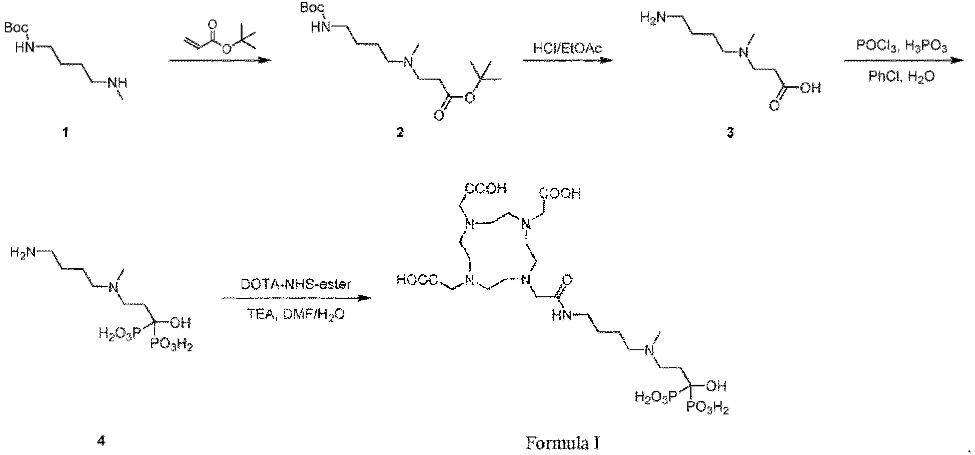

5. A preparation method of the radiolabeled compound according to claim 2 or 3, **characterized by** comprising: reacting a compound represented by Formula I with a radionuclide salt solution to obtain a radiolabeled compound represented by Formula II.

6. The preparation method according to claim 5, **characterized in**, when the radionuclide is ⁶⁸Ga, ¹⁷⁷Lu or ⁶⁴Cu, mixing a solution of the compound represented by Formula I, a sodium acetate solution, and a radionuclide salt solution evenly, adjusting pH value of the mixed solution, reacting, adjusting pH value again, sterilizing, and filtering to obtain the radiolabeled compound;
when the radionuclide is ²²⁵ Ac, mixing a solution of the compound represented by Formula I, a sodium citrate solution, a sodium ascorbate solution, and a radionuclide salt solution evenly, adjusting pH value of the mixed solution, reacting, adjusting pH value again, sterilizing, and filtering to obtain the radiolabeled compound;
when the radionuclide is ²¹¹ At, mixing a solution of the compound represented by Formula I, a sodium borate solution, and a radionuclide salt solution evenly, adjusting pH value of the mixed solution, reacting, adjusting pH value again, sterilizing, and filtering to obtain the radiolabeled compound.

7. The preparation method according to claim 6, **characterized in**, when the radionuclide is ⁶⁸Ga, adding 20-30 µg of the compound represented by Formula I to 0.8-1.4 ml of a sodium acetate solution with a concentration of 0.25M; then adding a ⁶⁸Ga salt solution with an activity of 20 mCi; mixing evenly, adjusting pH value of the mixed solution to 4-7, preferably 5; reacting at 80-100°C, preferably 95°C; the reaction time being 10-30min, preferably 15min; after the reaction, adjusting the pH value to 5; in which the solution of the compound represented by Formula I has a concentration of 1 mg/ml; and the ⁶⁸Ga salt solution has a concentration of 5mCi/ml to 10mCi/ml;
when the radionuclide is ¹⁷⁷Lu, adding 20-30 µg of the compound represented by Formula I to 0.8-1.4 ml of a sodium acetate solution with a concentration of 0.25M; then adding a ¹⁷⁷Lu salt solution with an activity of 2 mCi; mixing evenly, adjusting pH value of the mixed solution to 4-7, preferably 5; reacting at 80-100°C, preferably 95°C; the reaction time being 10-30min, preferably 15min; after the reaction, adjusting the pH value to 4.5; in which the solution of the compound represented by Formula I has a concentration of 1 mg/ml; and the ¹⁷⁷Lu salt solution has a concentration of 10mCi/ml to 20mCi/ml;
when the radionuclide is ²²⁵ Ac, adding 20-30 µg of the compound represented by Formula I to 0.8-1.4 ml of a sodium ascorbate solution with a concentration of 0.1M and 0.8-1.4 ml of a sodium citrate solution with a concentration of 0.1M; then adding a ²²⁵ Ac salt solution with an activity of 0.01 mCi; mixing evenly, adjusting pH value of the mixed solution to 4-7, preferably 5; reacting at 80-100°C, preferably 95°C; the reaction time being 10-30min, preferably 15min; after the reaction, adjusting the pH value to 4.5; in which the solution of the compound represented by Formula I has a concentration of 1 mg/ml; and the ²²⁵Ac salt solution has a concentration of 0.01mCi/ml to 0.02mCi/ml;
when the radionuclide is ⁶⁴Cu, adding 20-30 µg of the compound represented by Formula I to 0.8-1.4 ml of a sodium acetate solution with a concentration of 0.25M; then adding a ⁶⁴Cu salt solution with an activity of 5mCi; mixing evenly, adjusting pH value of the mixed solution to 4-7, preferably 5; reacting at 80-100°C, preferably 95°C; the reaction time being 10-30min, preferably 15min; after the reaction, adjusting the pH value to 4; in which the solution of the compound represented by Formula I has a concentration of 1 mg/ml; and the ⁶⁴Cu salt solution has a concentration of 5mCi/ml to 10mCi/ml;
when the radionuclide is ²¹¹ At, adding 20-30 µg of the compound represented by Formula I to 0.8-1.4 ml of a sodium borate solution with a concentration of 0.25M; then adding a ²¹¹At salt solution with an activity of 1mCi; mixing evenly, adjusting pH value of the mixed solution to 4-7, preferably 5; reacting at 80-100°C, preferably 95°C; the reaction time being 10-30min, preferably 15min; after the reaction, adjusting the pH value to 7; in which the solution of the compound represented by Formula I has a concentration of 1 mg/ml; and the ²¹¹At salt solution has a concentration of 1.0mCi/ml to 2.0mCi/ml.

8. Use of the compound represented by Formula I according to claim 1 in the manufacture of a medicament for both imaging and treating a metastatic bone tumor.

9. Use of the compound represented by Formula II according to claim 2 or 3 in the manufacture of a medicament for both imaging and treating a metastatic bone tumor.
